# EUROPEAN PATENT APPLICATION

(11) **EP 1 864 640 A1**
(43) Date of publication of application: **12.12.2007**
(21) Application number: 05822625.9
(22) Date of filing: 22.12.2005
(51) Int. Cl.: A61H 9/00, A61F 7/00

(54) **A HEALTH CARE AND PHYSICAL THERAPY DEVICE FOR GATHERING ENERGY**

(30) Priority: 18.02.2005 CN 200520054939 U
(71) Applicant: Fung, Kam Moon, Tai Po, Hong Kong (CN)
(72) Inventor: Fung, Kam Moon, Tai Po, Hong Kong (CN)
(74) Representative: Stuttard, Garry Philip
(86) International application number: PCT/CN2005/002286
(87) International publication number: WO 2006/086920

(57) **Abstract**

The present invention provides as healthy care device having a casing, having an inlet and an outlet on two opposite side thereof. The heater and the fan are installed inside the casing. The air guiding sleeve has a side connected to the outlet of the casing, the air guiding sleeve having another side with contact area to a patient's skin. The fan installed inside the casing near the inlet; a heater, near the air guiding sleeve and having structural connecting with the contact area to a patient's skin; and the energy concentrating element installing in the guiding sleeve. Applied the heater to heat the air induced by the fan, and through the outlet to the air guiding sleeve with the air guiding frame to the patient's skin surface. And the blowing opening is applied to exhaust the hot air. Then the gradually heating process to the skin of the patient is used to avoid the heating hurt of the patient. The safety and the reliability are reached. And the hot air can heat the energy concentrating element with corresponding energy wave coating inside the air guiding sleeve to generate the IR, magnetic energy, hot energy or other energy. The various kinds of energy can be focused to radiate the energy wave to enhance the human's self curing ability and blood circulation. The combination structure for physiotherapies is achieved. In addition, the temperature sensor and temperature control circuit let the temperature to be modulated. Then the application is safe and the reliable.

## Description

### Background of the Present Invention

### Field of Invention

The present invention relates to a healthy care and physical therapy device for gathering energy regarding the physiotherapy. Specially direct to relate to a healthy care device by a heating process.

### Description of Related Arts

On the recent days, there are applied methods on reducing inflammation or reducing painful reflection of human sense, and enhancing blood circulation by medicine or physiotherapy in the local or foreign medical institute. The general physiotherapy is by the IR (infrared ray) or magnetic material, which are good for reducing inflammation or reducing painful sense, and enhancing blood circulation. The equipment for the IR and the magnetic material are complex is expensive, and the effect not remarkable on the practical application. The physiotherapy in China includes acupuncture and moxibustion, surface hot pressing. The Chinese physiotherapies are more remarkable but they are dependent to the operator's skill. Accidents of hurting patients by piercing or burning could happen when the procedure is operated by un-skilled operator. The un-safe conditions are thought to be overcome.

### Summary of the Present Invention

The objective of the present invention is to provide a solution device to the un-safe condition for the physiotherapy and is to provided a simple structure with gradually heating the hurt part as a combining process by local Chinese and foreign method.

The present invention is to use a device comprise: a casing, the casing having an inlet and an outlet on two opposite side thereof; an air guiding sleeve, the air guiding sleeve having a blowing opening and connected to the casing, the air guiding sleeve having a contact area to a patient's skin; a fan, the fan installed inside the casing near the inlet; a heater, the heater installed in the casing near the air guiding sleeve and having structural connecting with the contact area to a patient's skin; and an energy concentrating element, the energy concentrating element installing in the guiding sleeve.

A temperature sensor installed on the air guiding sleeve near the contact area to a patient's skin; and a temperature control circuit connecting to the temperature sensor, wherein the temperature control circuit connected to the heater and the fan, whereby the temperature sensor measures the temperature number to modulate the power of the fan and the heater.

The blowing opening is installed on one place selected from the side wall of the air guiding sleeve, and the connecting portion of the air guiding sleeve with the outlet of the casing.

The air guiding sleeve has a through hole on the near side of the contact area to a patient's skin.

The energy concentrating element is a circle structure made of the material selected the group of copper, silver, and carbon fiber.

The heater is made of selected from the group of heating wire, heating tube, and the IR tube.

The casing further comprising a handle for operation; and a switch on the handle.

The air guiding sleeve further comprising a air guiding frame; and a through hole is placed on the surface of the guiding frame.

The air guiding sleeve further comprising a frame structure for scraping; and a tube shape is placed on the frame structure for scraping.

On comparing to the prior art, the present invention as healthy care device apply the heater to heat the air induced by the fan, and through the outlet to the air guiding sleeve with the air guiding frame to the patient's skin surface. And the blowing opening is applied to exhaust the hot air. Then the gradually heating process to the skin of the patient is used to avoid the heating hurt of the patient. The safety and the reliability are reached. And the hot air can heat the energy concentrating element inside the air guiding sleeve to generate the IR, magnetic energy, hot energy or ion energy. The various kinds of energy can be focused to radiate the energy wave to enhance the human's self curing ability and blood circulation. The combination of Chinese and foreign curing method is achieved. In addition, the disturbed immunization system, internal organs and the circulation system in body can be improved. And a frame structure can be add on the air blowing opening, the frame structure can be switched to change the hole size to change the amount of the air flow rate. Then the temperature of the outlet airflow can be change as a function purpose.

One or part or all of these and other features and advantages of the present invention will become readily apparent to those skilled in this art from the following description wherein there is shown and described a preferred embodiment of this invention, simply by way of illustration of one of the modes best suited to carry out the invention. As it will be realized, the invention is capable of different embodiments, and its several details are capable of modifications in various, obvious aspects all without departing from the invention. Accordingly, the drawings and descriptions will be regarded as illustrative in nature and not as restrictive.

### Brief Description of the Drawings

Fig. 1 illustrates an embodiment of a healthy care device as a cross-section view for the first structure of the present invention;
Fig. 2 illustrates an embodiment of a healthy care device as a cross-section view for the second structure of the present invention;
Fig. 3 illustrates a air guiding frame in the Fig. 2;
Fig. 4 illustrates a side view on A-direction in the Fig. 3;
Fig. 5 illustrates an embodiment of a frame structure for scraping, and the frame structure for scraping is instead of air guiding frame,
Fig. 6 illustrates a side view on B-direction in the Fig. 5;
Fig. 7 illustrates an embodiment of a healthy care device as a cross-section view for the third structure of the present invention; and
Fig. 8 illustrates an embodiment of a circuit diagram for Figs.1-2.

### Detailed Description of the Preferred Embodiments

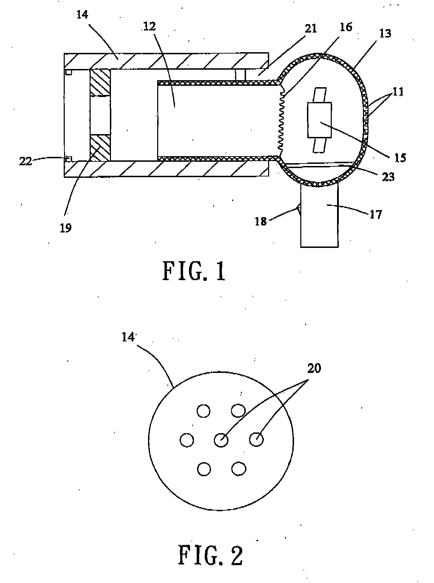

Referring to FIGs. 1-8, there are the embodiments of the present invention. The below are the descriptions in detail.

For the described in the Fig. 1, there is a embodiment of the present invention, it comprising: a casing 13, the casing having an inlet 11 and an outlet 12 on two opposite side thereof; an air guiding sleeve 14, the air guiding sleeve 14 (with void space inside) having a blowing opening 21 and connected to the outlet 12 of the casing 13. A fan 15 and a heater 16 are installed inside the casing 13, and the casing 13 can be a cylinder shape. Wherein the heater 16 is made of selected from the group of heating wire, heating tube, and the IR tube. The outside current source can provide power to the heater 16 and the fan 15. The casing 13 can further comprise a handle 17 for operation and a switch 18 installed on the handle 17. The fan 15 induce the outside air into the casing 13, and using the heating 16 to heat the air to exhaust to a outlet 12 to reach the skin of a patient.

An energy concentrating element 19, the energy concentrating element 19 installing in the guiding sleeve 14. The energy concentrating element 19 is a circle structure made of the material selected the group of copper, silver, and carbon fiber. The hot air can heat the energy concentrating element inside the air guiding sleeve to generate the IR, magnetic energy, hot energy or ion energy. the various kinds of energy can be focused to radiate the energy wave to enhance the human's curing ability. In the embodiment the air guiding sleeve 14 is a void cylinder and installed on the outlet 12 of the casing 13. The air guiding sleeve 14 can have a plurality of through holes 20 on the near side of the contact area to a patient's skin. The blowing opening 21 is installed on one place selected from the sidewall of the air guiding sleeve 14, and the connecting portion of the air guiding sleeve 14 with the outlet of the casing 13. The blowing opening 21 is for hot air exited

When in the applied period, the air guiding sleeve 14 having a contact area to a patient's skin. When the power is turned on, the fan and the heater are activated. The outside air is induced into the casing 13 to heat by using the outlet 12 and the air guiding sleeve 14 combining the air guiding frame 25 or the frame structure for scraping 26 to reach the skin of the patient. And the hot air is outgoing by the outlet 12 to get the gradually heating property on the air guiding sleeve 14. The patient's skin can gradually heat to avoid the un-suitable condition and to enhance the human's lymph circulation and blood circulation to cure the rheumatism related disease or body joint inflammatory response. Accordingly the hot air can heat the energy concentrating element 19 inside the air guiding sleeve 14 to generate the IR, magnetic energy, hot energy or ion energy, the various kinds of energy can be focused to radiate the energy wave to get the proper process of self curing method.

For the safety object achieved, a temperature sensor 22 installed on the air guiding sleeve 14 near the contact area to a patient's skin; and a temperature control circuit 23 (inside the casing 13) is connected to the temperature sensor 22. Wherein the temperature control circuit 23 connected to the heater 16 and the fan 15, whereby the temperature sensor 22 measures the temperature number to modulate the power of the fan 15 and the heater 16. Then the gradually heating process to the skin of the patient is used to avoid the heating hurt of the patient.

For the described in the Fig. 8, the temperature control circuit is displayed by a layout diagram. The temperature sensors switch is the S4; the R8 and R9 are two initial number resistors set on the high temperature and the low temperature each thereof. In addition, the temperature control circuit can be extended to include the R4-R7, and the temperature sensor switch S3 and the capacitor C. Thus the two-direction controllable switch S1 in circuit can be formed to execute the temperature control function. But the other related circuit can be applied to use the above similar function.

The process for the temperature controlling can be in the following steps, at the status of the temp lower than the related set temp, the related sensor switch is at the breaking state. Then the R4-R7 is generating a related phase signal to activate the sensor switch S3, and at the same period the two-direction controllable sensor S1 is activated to link the circuit. The AC current is activated on a positive half period, the capacitor C to charge; but at a negative half period current activating, the capacitor C to discharge. In the operation the current activates the switch S1 for current is on linked in the positive to negative period. At the temperature lower than the set amount, the switch S4 is on linked rather than the switch S3 is activated by the side circuit line to be at break status. And the S1 is not activated to be at a status not to link the current circuit to the heater. And the natural dissipation is formed to low down the temperature. And at the temperature is lowing down to a number of amount, the switch S4 is changed from the on status to the break status to let the heating process to formed to make the switch S3, S 1 being on status for heating.

For the described in the Fig. 7, an applied embodiment is shown as the healthy care device. The air guiding sleeve 14 has a side connected to the outlet 12 of the casing 13, the air guiding sleeve 14 having another side with contact area to a patient's skin. And the energy concentrating element 19 can be installed on the outside range of the air guiding sleeve 14. In addition, the protection frame 24 can be installed on the air guiding sleeve 14 for avoiding of the hot hurting from the air guiding sleeve 14.

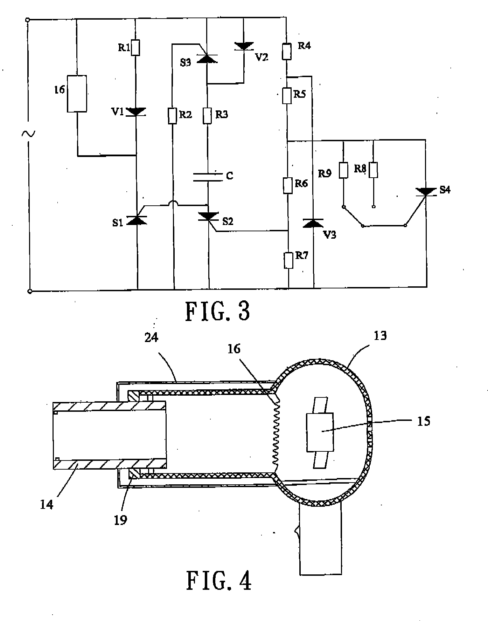

For the described in the Fig. 2, an applied embodiment shown as the air guiding sleeve 14 further comprising a air guiding frame 25 (Fig. 3-4); and a plurality of through holes 20 are uniformly placed on the surface of the air guiding frame 25. The air guiding frame 25 can be made of the material of the carbon fiber and the metal. The enhanced effect can be achieved by the air guiding frame 25. The air guiding sleeve 14 further comprising a frame structure for scraping 26 (Fig. 5-6). The scraping method can be applied on the skin for curing. And a tube shape is placed on the frame structure for scraping 26 or the end shape of the frame structure for scraping 26 is a arc shape. The frame structure for scraping 26 can be made of the material of the carbon fiber and the metal. The above described of the air guiding frame 25 can be structurally replaced by the frame structure for scraping 26.

Although the invention has been described and illustrated with reference to specific illustrative embodiments thereof, it is not intended that the invention be limited to those illustrative embodiments. Those skilled in the art will recognize that variations and modifications can be made without departing from the spirit of the invention. It is therefore intended to include within the invention all such variations and modifications which fall within the scope of the appended claims and equivalents thereof. One skilled in the art will understand that the embodiment of the present invention as shown in the drawings and described above is exemplary only and not intended to be limiting.

The foregoing description of the preferred embodiment of the present invention has been presented for purposes of illustration and description. It is not intended to be exhaustive or to limit the invention to the precise form or to exemplary embodiments disclosed. Accordingly, the foregoing description should be regarded as illustrative rather than restrictive. Obviously, many modifications and variations will be apparent to practitioners skilled in this art. The embodiments are chosen and described in order to best explain the principles of the invention and its best mode practical application, thereby to enable persons skilled in the art to understand the invention for various embodiments and with various modifications as are suited to the particular use or implementation contemplated. It is intended that the scope of the invention be defined by the claims appended hereto and their equivalents in which all terms are meant in their broadest reasonable sense unless otherwise indicated. It should be appreciated that variations may be made in the embodiments described by persons skilled in the art without departing from the scope of the present invention as defined by the following claims. Moreover, no element and component in the present disclosure is intended to be dedicated to the public regardless of whether the element or component is explicitly recited in the following claims.

## Claims

1. A health care and physical therapy device for gathering energy, comprising:
a housing having an air inlet and an air outlet at both ends thereof;
a hollow air-directing sheath having an exhaust port, one end of the air-directing sheath being disposed at the air outlet of the housing, and the other end of the air-directing sheath having a contact area to a patient's skin;
a fan motor being installed inside the housing;
a heating element being installed inside the housing; and
an energy-gathering member being installed in the directing sheath.

2. The health care and physical therapy device as claimed in claim 1 further comprising:
a temperature sensor being installed on the air-directing sheath near the contact area to a patient's skin; and
a temperature controlling circuit being disposed in the housing and connected to the temperature sensor.

3. The health care and physical therapy device as claimed in claim 2, wherein the temperature controlling circuit is connected to the heating element and the fan motor, and the temperature sensor measures temperature to modulate a power of the fan motor or the heating element.

4. The health care and physical therapy device as claimed in claim 1, 2 or 3, wherein the exhaust port is disposed in a side wall of the air-directing sheath, or a connecting portion of the air-directing sheath with the air outlet of the housing.

5. The health care and physical therapy device as claimed in claim 4, wherein the air-directing sheath is formed with at least one through hole on the near side of the contact area to a patient's skin.

6. The health care and physical therapy device as claimed in claim 5, wherein the energy-gathering member is a circle structure made of copper, or silver.

7. The health care and physical therapy device as claimed in claim 6, wherein the heating element is a heating wire or a heating tube.

8. The health care and physical therapy device as claimed in claim 7, wherein the housing further includes a handle for operation and a switch on the handle.
